# EUROPÄISCHE PATENTSCHRIFT

(11) **EP 2 106 761 B1**
(45) Veröffentlichungstag und Bekanntmachung des Hinweises auf die Patenterteilung: **06.04.2011**
(21) Anmeldenummer: 08019603.3
(22) Anmeldetag: 10.11.2008
(51) Int. Cl.: A61B 17/70

(54) **Implantat zum Beabstanden von benachbarten Knochen einer Wirbelsäule**
Implant for separating neighbouring bones in a spine
Implant d'éloignement d'os contigus d'une colonne vertébrale

(30) Priorität: 09.11.2007 DE 102007053884
(43) Veröffentlichungstag der Anmeldung: 07.10.2009
(73) Patentinhaber: Unger, Michael, 78573 Wurmlingen (DE)
(72) Erfinder: Unger, Michael, 78573 Wurmlingen (DE); Streng, Jürgen, Dr.-Ing., 78532 Tuttlingen (DE)
(74) Vertreter: Weiss, Peter

(56) Entgegenhaltungen:
- WO-A-2006/113080
- US-A1- 2005 203 512
- US-A1- 2006 084 988
- US-A1- 2006 184 247
- US-B1- 6 235 030

## Beschreibung

Die Erfindung betrifft ein Implantat gemäß dem Oberbegriff des Patentanspruchs 1.

Die Wirbelsäule eines Erwachsenen ist in der Regel doppelt s-förmig gekrümmt, sodass sie quasi als federnder Stab vor allem dem aufrecht gehenden Menschen zu einem elastischen Gangbild verhilft, wobei bekanntlich die nach vorne konvexe Krümmung im Hals- und Lendenwirbelbereich als Lordose und die nach hinten konvexe Krümmung im Brust- und Sakralbereich als Kyphose bezeichnet wird. Nachdem die Wirbelsäule als Achse des Menschen den Kopf, die Rippen und die oberen Extremitäten tragen muss, ist sie des öfteren die Ursache von Rückenschmerzen, da deren einzelne Knochenabschnitte je nach Belastung und Verschleiß aneinander reiben können, sodass dadurch das elastische Gangbild nicht mehr aufrechterhalten werden kann.

Insbesondere bei den so genannten Dornfortsätzen der Wirbelsäule, die ausgehend von den Wirbelbögen der einzelnen Wirbelkörper rückenwärts gerichtet sind und als Ansatzstellen für Bänder und Muskeln dienen, kann sich krankheitsbedingt jeweils der Abstand benachbarter Dornfortsätze zum Nachteil des Patienten merklich verringern, wodurch erhebliche Schmerzen auftreten können, wenn sich der Patient bewegt, d.h. wenn relative Bewegungen zwischen derartigen Knochenabschnitten der Wirbelsäule auftreten.

Es gibt bereits in der orthopädischen Chirurgie eine Reihe von Verfahren zur Linderung von derartigen Rückenproblemen, die sich vor allem mit einem Stabilisieren der betroffenen Wirbel befassen, was z. B. durch ein mechanisches Verbinden der Wirbel miteinander gelöst werden kann. Ferner sind Verfahren bekannt, bei denen die rückenwärts gerichteten Dornfortsätze einer Wirbelsäule mit Hilfe eines Implantats beabstandet werden, damit diese sich nicht mehr berühren bzw. aneinander reiben können. So ist beispielsweise in der US 2004/0193159 A1 ein derartiges Verfahren beschrieben, bei dem die Stabilisierung eines Abstands zwischen den Dornfortsätzen benachbarter Wirbelkörper mit Hilfe eines quasi zusammen- schraubbaren Implantats erreicht werden soll, das relativ aufwendig jeweils zwischen zwei benachbarten Dornfortsätzen chirurgisch eingesetzt wird. Dazu kommt, dass bei allen bekannten derartigen Verfahren mit eingesetzten Implantaten die hinterher erzielten Bewegungsmöglichkeiten des jeweiligen Patienten mehr oder weniger begrenzt bleiben.

Beispielsweise offenbart die US 2005/0203512 A1 ein Implantat zum Beabstanden von Wirbelkörpern. Dieses weist ein Seitenteil, einen Mittelteil und einen Verformungsabschnitt auf. Der Verformungsabschnitt ist zur Implantierung des Implantates so ausgebildet, dass er eine Verlängerung des Mittelabschnitts darstellt und durch die Wirbelkörper hindurch gesteckt werden kann. Wenn das Implantat positioniert ist, kann der Verformungsabschnitt zu einem weiteren Seitenteil verformt werden, das das Implantat zwischen den Wirbelkörpern hält.

Ein vergleichbares Implantat offenbart die US 6,235,030 B1. Hier werden die Halteplatten an ein Mittelteil gesteckt, das bereits zwischen die Wirbelkörper bzw. die Dornfortsätze eingebracht ist.

Desweiteren offenbart die WO 2006/113080 A2 ein Implantat, das nach dem Prinzip einer Blind-Niete verformbar ist. Die Verformungsabschnitte weisen Sollbruchstellen auf, an denen die Verformungsabschnitte abknicken und so Halteflansche ausbilden.

Auch die US 2006/0184247 A1 offenbart ein Implantat zum Beabstanden von Wirbelknochen. Dieses weist Verformungsabschnitte auf. Die Verformungsabschnitte können von aussen so zusammengedrückt werden, dass sie Halteflansche ausbilden.

Auch die US 2006/0084988 A1 offenbart ein Implantat, das nach dem Prinzip einer Blind-Niete verformt werden kann. Dabei weitet sich der ganze Implantatkörper, auch der mittlere Bereich, der zwischen den Knochen liegt, auf. Demgegenüber liegt der Erfindung die Aufgabe zugrunde, ein Implantat anzugeben zum Beabstanden von benachbarten Knochen einer Wirbelsäule, insbesondere von den Wirbelbögen der Wirbelkörper ausgehenden, rückenwärts gerichteten Dornfortsätze zu schaffen, das minimalinvasiv implantierbar ist und gleichzeitig die Freiheitsgrade der Wirbelsäule, wie Vor- und Rückbewegung, Seitenneigung und Drehung ermöglicht.

Die Aufgabe wird durch die Merkmale der kennzeichnenden Teils des Patentanspruchs 1 gelöst.

Ein relativ schlanker zylindrischer Implantatkörper als Ausgangsform wird zwischen die zu beabstandenden Dornfortsätze implantiert und anschließend nach der Art einer Niete an den Dornfortsätzen fixiert. Nach Maßgabe der vorliegenden Erfindung ist es dabei besonders vorteilhaft, wenn für die Ausgangsform des Implantats ein zylindrischer Implantatkörper mit einem mittleren Dämpfungsabschnitt, an dem sich beidseitig je ein Verformungsabschnitt anschließt, verwendet wird, wobei die Verformungsabschnitte nach der Implantierung zum Fixieren des Implantats an benachbarten Dornfortsätzen jeweils zu Halteflanschen verformt werden, die an den Dornfortsätzen lediglich anliegen und nicht mit diesen verschraubt werden.

Hierbei ist es zweckmäßig, wenn der zylindrische, vorzugsweise im Querschnitt bspw. kreisförmige Implantatkörper mit einer Längsachse hergestellt und in deren Mittelbereich ein Röhrchen, vorzugsweise aus Titan, eingesetzt wird, dessen Länge dem maximalen äußeren Abstand zwischen den verformten Halteflanschen im implantierten Endzustand entspricht. Das Titanröhrchen wirkt gleichzeitig als Indikator für die Erkennung des Implantats auf Röntgenbildern.

Ferner ist es dabei zweckmäßig, wenn in die hohle Längsachse mit dem eingesetzten Röhrchen des Implantatkörpers ein Stift, vorzugsweise ebenfalls aus Titan, gesteckt wird, dessen Längserstreckung auf der einen Endseite des stangenförmigen Implantatkörpers mit dieser praktisch abschließt und auf der anderen Endseite aus der hohlen Längsachse herausragt.

Das Blind-Niet Prinzip kann in Weiterbildung der vorliegenden Erfindung auf einfache Weise dadurch erreicht werden, dass der Stift auf der abschließenden Endseite des stangenförmigen Implantatkörpers mit einer an dessen Stirnfläche angreifenden Konterscheibe versehen wird, wogegen auf das aus der Längsachse des Implantatkörpers herausragende Trumm des Stiftes eine auf diese Endseite an der Stirnseite des Implantatkörpers angreifende Druckscheibe vorerst locker aufgeschoben wird.

Hierbei ist es von Vorteil, wenn die Einbringung des zylindrischen Implantatkörpers in den zu beabstandenden Bereich der Wirbelsäule eines Patienten mit Hilfe eines röhrenförmigen Implantierungsinstruments durchgeführt wird, dass in seinem Frontbereich den zylindrischen Implantatkörper sowie einen Stempel aufweist, mit dem der Implantatkörper aus dem röhrenförmigen Instrument in eine gewünschte Position in der Wirbelsäule gebracht wird, wobei der zylindrische Implantatkörper hierbei derart in den Zwischenraum zweier zu beabstandenden Dornfortsätze einer Wirbelsäule eingebracht wird, dass sich der mittlere Dämpfungsabschnitt des Implantatkörpers in dem Zwischenraum befindet und diesen voll ausfüllt, wogegen sich die Verformungsabschnitte beidseitig der Dornfortsätze erstrecken.

Zur Fixierung des Implantats ist es dabei zweckmäßig, wenn das freie Trumm des aus dem implantierten zylindrischen Implantatskörpers herausragenden Stifts mit einer Implantatfixierungszange erfasst und vorzugsweise durch Zusammendrücken deren Zangengriffe oder eines anderen mechanischen Verfahren zum Aufbau von Zugkräften die Länge des Stiftes derartig verkürzt wird, dass eine ausreichende Kraft auf die Konter- und Druckscheibe in den Endbereichen der Verformungsabschnitte ausgeübt wird, damit diese zu den Halteflanschen verformt werden. Hierbei ist es zweckmäßig, dass nach der Verkürzung der Längserstreckung des zylindrischen Implantatkörpers und der dabei erzielten Verformung der plastisch flexiblen Verformungsabschnitte zu Halteflanschen, das freie Trumm des Stiftes an der richtigen Stelle zu kappen und dabei gleichzeitig diesen Endbereich zu befestigen, sodass die eingestellte Gesamtlänge des Stiftes und somit die Gesamtbreite des im Endzustand vorliegenden Implantats gewährleistet ist.

Dieses Abtrennen des Stiftes kann auch noch dadurch erleichtert werden, indem das freie Trumm des Stiftes zur Fixierung der Breite eines eingesetzten Implantates mit einer Sollbruchstelle versehen wird, an der nach der Verformung der Verformungsabschnitte des Implantatkörpers das Trumm des Stiftes gekappt wird.

Als Implantat selbst eignet sich vor allem eine als zylindrischer Implantatkörper ausgebildete Ausgangsform, die mit einem mittleren Dämpfungsabschnitt und beidseitig zu diesem mit jeweils einem Verformungsabschnitt ausgestaltet ist, wobei die Verformungsabschnitte derart plastisch flexibel beschaffen sind, dass sie nach einer Implantierung des Ausgangskörpers zwischen benachbarte Dornfortsätze zum Fixieren des Dämpfungsabschnitts an diesen jeweils zu Halteflanschen verformbar sind, d. h. die Halteflansche legen sich elastisch an die Dornfortsätze an ohne mit diesen verschraubt werden zu müssen.

Weitere Einzelheiten und Vorteile der Erfindung ergeben sich aus der nachfolgenden Beschreibung mehrer Ausführungsbeispiele in Verbindung mit der Zeichnung; darin zeigt:
- Fig.1: eine schräge Draufsicht auf zwei benachbarte Wirbelkörper einer Wirbelsäule;
- Fig.2: eine schematische Darstellung der Ausgangsform eines zylindrischen Implantatkörpers als erstes Ausführungsbeispiel in einem vergrößerten Maßstab;
- Fig.3: den Implantatkörper gemäß Fig. 2 in seiner implantierten und nach dem Blind-Niet-Prinzip fixierten Lage zwischen zwei benachbarten Dornfortsätzen der beiden Wirbelkörper gemäß der Schnittlinie III - III in Fig. 1;
- Fig.4: eine schematische Darstellung eines röhrenförmigen Implantationsinstruments zum Einbringen eines zylindrischen Implantatkörpers gemäß Fig. 5 in den zu beabstandenden Bereich der Dornfortsätze gemäß Fig. 1;
- Fig.5: eine im Maßstab vergrößerte schematische Darstellung der Ausgangsform eines zylindrischen Implantatkörpers mit einer ellipsoiden Spitze und einer außenliegenden Konter- und Druckscheibe als zweites Ausführungsbeispiel;
- Fig.6: eine schematische Darstellung der Ausgangsform eines zylindrischen Implantatkörpers analog zu Fig. 5, jedoch mit einer eingebetteten Konter- und Drucksscheibe als drittes Ausführungsbeispiel und
- Fig.7: den Implantatkörper gemäß Fig. 6 in seiner implantierten Form, jedoch ohne Dornfortsätze dargestellt.

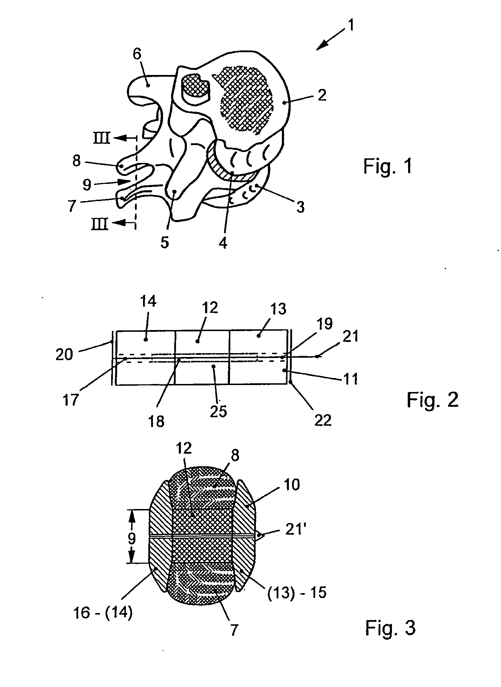

Der in Fig. 1 in schräger Draufsicht gezeigte Abschnitt einer Wirbelsäule 1 besitzt zwei benachbarte Wirbelkörper 2 und 3, zwischen denen sich eine Bandscheibe 4 befindet. An den Wirbelbögen der beiden Wirbelkörper 2 und 3 erstrecken sich rückenwärts neben Querfortsätzen 5 und 6 (nur beim Wirbelkörper 1 angegeben) jeweils ein Dornfortsatz 7 und 8, deren Zwischenraum 9 durch ein Implantat 10 (Fig. 3) in einer ersten Ausführungsform beabstandet werden soll bzw. gemäß Fig. 3 ist.

In Fig. 2 ist die Ausgangsform des Implantats 10 gemäß Fig. 3 als zylindrischer Implantatkörper 11 vergrößert schematisch dargestellt, der einen mittleren Dämpfungsabschnitt 12 aus einem dauerelastischen Kunststoffmaterial z.B. einem Thermoplasten oder aus einer Materialkombination von Kunststoffen und metallischen Werkstoffen oder Legierungen wie z.B. Titan, Nitinol, etc. sowie beidseitig Verformungsabschnitte 13 und 14 aus einem plastisch verformbaren, flexiblen, oder dauerelastischen Material besitzt, die - wie in Fig. 3 dargestellt - zu Halteflanschen 15 und 16 verformt worden sind.

Der zylindrische Implantatkörper 11 ist im Querschnitt kreisförmig ausgebildet und besitzt eine hohle Längsachse 17, in deren Mittelbereich ein Röhrchen 18 eingesetzt ist, das in vorliegendem Ausführungsbeispiel aus Titan besteht, sodass dieses einerseits als Indikator für die Erkennung des Implantats 10 auf Röntgenbildern dient und andererseits als Führung eines Titanstifts 19. Die Länge des Röhrchens 18 entspricht dem maximalen äußeren Abstand zwischen den verformten Halteflanschen 15 und 16 im implantierten Endzustand des Implantats 10 (Fig. 3).

Der in das Röhrchen 18 des zylindrischen Implantatkörpers 11 gesteckte Titanstift 19 schließt auf der linken Endseite mit einer Konterscheibe 20 ab, wogegen dieser auf der rechten Endseite aus der Längsachse 17 mit einem Trumm 21 herausragt, auf das eine Druckscheibe 22 aufgefädelt ist.

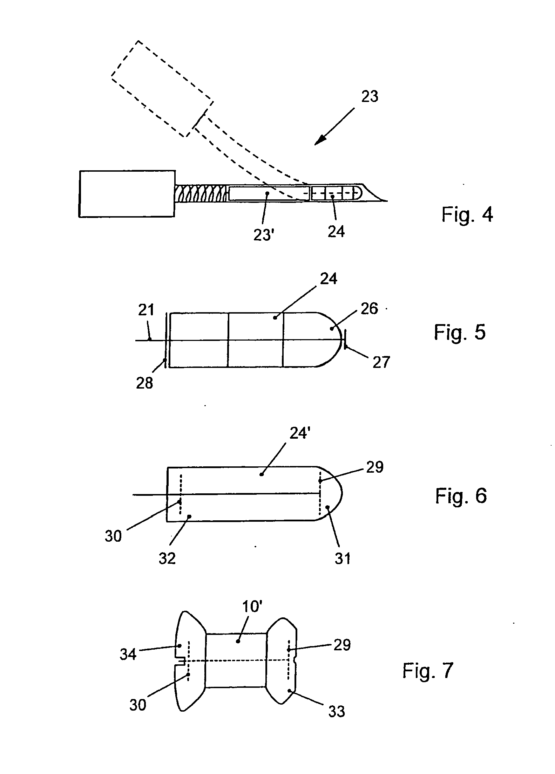

Zum Einbringen des zylindrischen Implantatkörpers 11 in den zu beabstandenden Bereich des Wirbelkörperabschnitts 1, hier der Abstand 9 in Fig. 1 und in Fig. 3, dient ein röhrenförmiges Implantierungsinstrument 23, das in Fig. 4 schematisch dargestellt ist. Im Frontbereich des Instruments 23 ist der zylindrische Implantatkörper 11 bzw. der in Fig. 6 gezeigten zylindrischen Implantatkörper 24 als zweite Ausgangsform positioniert. Dahinter befindet sich ein Stempel 23', mit dem die Ausgangsformen der Implantate aus dem röhrenförmigen Instrument 23 in die gewünschte Position in dem Wirbelsäulenabschnitt 1 gebracht werden können.

Zur Fixierung des Implantats 10 an den Dornfortsätzen 7 und 8 dient eine nicht näher dargestellte Implantatfixierungszange, welche separat oder in Kombination mit dem Impantierungsinstrument 23 - wie in Fig.4 gestrichelt angedeutet - zur Anwendung kommen kann. Mit einer derartigen Zange wird das freie Trumm 21 gemäß Fig. 2 erfasst und die Länge des Titanstiftes 19 durch ein Zusammendrücken deren Zangengriffe derart verkürzt, dass eine ausreichende Kraft auf die Konter- und Duckscheibe 20 und 22 in den Endbereichen der unverformbaren Verformungsabschnitte 13 und 14 erzeugt wird, um diese dann zu den plastisch oder elastisch verformten Halteflanschen 15 und 16 zu verformen.

Damit die Gesamtbreite des im Endzustand implantierten Implantats 10 festgelegt ist, kann die Verkürzungsstrecke des freien Trumms 21 des Titanstiftes 19 durch eine Sollbruchstelle 25 an diesem begrenzt werden. Alternativ kann ohne Verwendung einer Sollbruchstelle der zentrale Titanstift auf eine beliebige Länge verkürzt und durch mechanische Verformung in seiner Verkürzungsposition arretiert werden. Anschliessend kann das überstehende Stück des freien Trumms mechanisch und spanfrei abgetrennt werden.

Neben der ersten Ausführungsform für einen zylindrischen Implantatkörper 11 gemäß Fig. 2 sind auch weitere Ausführungsformen möglich. So ist in Fig. 5 als zweite Ausführungsform der bereits erwähnte Implantatkörper 24 schematisch dargestellt, dessen einer Verformungsabschnitt 26 sich in Richtung seines freien Endes verjüngt, d. h. als Abschnitt eines Ellipsoids ausgebildet ist, wobei dann der Durchmesser der mit 27 bezeichneten Konterscheibe im Bereich der ellipsoiden Verjüngung entsprechend kleiner ausgebildet ist als der Durchmesser der dazugehörigen mit 28 bezeichneten Druckscheibe.

Schließlich ist in den Fig. 6 schematisch eine Ausgangsform für einen Implantatkörper 24' und in Fig. 7 ein daraus geformtes Implantat 10' als drittes Ausführungsbeispiel dargestellt, bei welchem die mit 29 und 30 bezeichneten Konter- und Druckscheiben innerhalb der mit 31 und 32 bezeichneten Verformungsabschnitten angeordnet sind, welche dann im implantierten Endzustand zu Halteflanschen 33 bzw. 34 führen.

Das Verfahren zum Einbringen eines der erfindungsgemäßen Implantate in die Wirbelsäule eines Patienten erfolgt nach folgender Operationsmethode:

Zuerst wird seitlich der Wirbelsäule auf dem Rücken des Patienten ein Zugang zum betroffenen Abschnitt der Wirbelsäule durch einen kleinen Schnitt und gegebenenfalls durch eine notwendige Freipräparierung geschaffen. Sodann wird das Implantierungsinstrument 23 mit z.B. dem zylindrischen Implantatkörper 11 mittels seiner scharfen Spitze an die Rückenöffnung angesetzt, wobei durch zumindest eine seitliche Aufdickung oder exzentrischen Anordnung der zylindrischen, insbesondere röhrenförmige Form ein Aufspreizen der zu beabstandeten Dornfortsätze möglich ist. Die Aufspreizung kann auch durch eine ovale Verdickung und ein entsprechendes Verdrehen oder durch eine im Durchmesser zunehmende Verdickung des Endbereichs des röhrenförmigen Implantierungsinstruments 23 erfolgen. Die präzise Einbringung des Implantatkörpers 11 in den Zwischenraum 9 der benachbarten Dornfortsätze 7 und 8 der Wirbelsäule 1 erfolgt dann durch den Stempel 23', wobei dieser so ausgelegt ist, dass dieser den Titanstift 19 des zylindrischen Implantatkörpers 11 nicht behindert. Hierbei wird der Implantatkörper 11 durch den Stempel 23' aus dem röhrenförmigen Endabschnitt des Instruments 23 herausgedrückt während gleichzeitig das gesamte Instrument 23 zurückgezogen wird.

Sodann wird mit der Implantatfixierungszange, wie bei einer Nietzange, der Titanstift am Trumm 21 erfasst und dieses eingezogen, sodass Druck auf beide Scheiben 20 und 22 ausgeübt wird und sich dadurch bei gleichzeitiger Verkürzung des Abstandes zwischen den äußeren Konturen der Verformungsabschnitte 13 und 14, diese zu den Halteflanschen 15 und 16 verformen. Nach der gewünschten Verkürzung und der damit erzielten Aufweitung der plastisch flexiblen Außenbereiche des Implantatskörpers 11 wird der Titanstift 19 z. B. an der Sollbruchstelle 25 gekappt. Dabei wird der Titanstift 19 derart bei 21' verformt bzw. festgelegt, dass seine eingestellte Lage fest bleibt. Vorteilhaft wird dabei durch die Spannung und die Rückformkräfte der flexiblen Außenbereiche das eingesetzte Implantat 10 formstabil und präzise positioniert, sodass der Patient wieder seine drei Freiheitsgrade bezüglich seiner Rückenbewegung erhält, d. h. sich schmerzfrei wie gewohnt bewegen kann.

## Patentansprüche

1. Implantat zum Beabstanden von benachbarten Knochen einer Wirbelsäule, insbesondere den von Wirbelbögen ausgehenden, rückenwärts gerichteten Dornfortsätzen, wobei das Implantat (10; 10') in seiner Ausgangsform als zylindrischer Implantatkörper (11; 24; 24') mit einem mittleren Dämpfungsabschnitt (12) und sich beidseitig an diesen anschließenden Verformungsabschnitten (13, 14; 31, 32) ausgebildet ist, wobei die Verformungsabschnitte (13, 14; 31; 32) derart beschaffen sind, dass sie nach einer Implantierung des Ausgangskörpers (11; 24; 24') zwischen benachbarte Dornfortsätze (7; 8) zum Fixieren des Dämpfungsabschnitts (12) an den Dornfortsätzen (7; 8) jeweils zu Halteflanschen (15, 16; 33, 34) nach dem Prinzip einer Blind-Niete verformbar sind, und der Implantatkörper eine hohle Längsachse (17) aufweist wobei in die hohle Längsachse (17) mit dem eingesetzten Röhrchen (18) des Implantatkörpers (11, 24) ein Titanstift (19) gesteckt ist, dessen Länge derartig verkürzbar ist, dass eine ausreichende Kraft auf eine Konter- und Druckscheibe (22, 28, 29, 30) in Endbereichen der Verformungsabschnitte (13, 14) ausübbar ist, damit diese zu den Halteflanschen (15,16) verformbar sind und sich elastisch an die Dornfortsätze (7, 8) anlegen,
**dadurch gekennzeichnet,**
**dass** in deren Mittelbereich der Längsaches (17) ein Titanröhrchen (18) eingesetzt ist, dessen Länge dem maximalen äusseren Abstand zwischen den verformten Halteflanschen (15, 16) im implantierten Endzustand des Implantats (10) entspricht.

2. Implantat nach Anspruch 1, **dadurch gekennzeichnet, dass** der zylindrische Implantatkörper (11; 24; 24') vorzugsweise im Querschnitt zylindrisch, in dem kreisförmig oder vieleckartig ausgebildet ist , und dass die Längenerstreckung des Stiftes (19) auf der einen Endseite des Implantatkörpers (11; 24) mit dieser praktisch abschließt und auf der anderen Endseite aus der hohlen Längsachse (17) mit einem Trumm (21) herausragt.

3. Implantat nach Anspruch 2, **dadurch gekennzeichnet, dass** an dem Stift (19) an der abschließenden Endseite des zylindrischen Implantatkörpers (11; 24) eine an dessen Stirnfläche anliegende Konterscheibe (20; 27) befestigt ist, wogegen auf das aus der Längsachse (17) des Implantatkörpers (11; 24) herausragende Trumm (21) des Stiftes (19) eine auf dieser Endseite an die Stirnseite des Implantatkörpers (11; 24) anliegende Druckscheibe (22; 28) auf dem Trumm (21) verschiebbar angeordnet ist.

4. Implantat nach Anspruch 3, **gekennzeichnet durch** den zylindrischen Implantatskörper (11; 24; 24'), geeignet zur Einbringung in den zu beabstandenen Bereich (9) der Wirbelsäule (1) eines Patienten mit einem röhrenförmigen Implantierungsinstrument (23), das in seinem Frontbereich den zylindrischen Implantatkörper (11; 24; 24') sowie einen Stempel (23') aufweist, mit dem der Implantatkörper aus dem röhrenförmigen Implantierungsinstrument (23) in eine gewünschte Position in der Wirbelsäule (1) einbringbar ist.

5. Implantat nach Anspruch 4, **dadurch gekennzeichnet, dass** der zylindrische Implantatkörper (11; 24; 24') derart in den Zwischenraum (9) zweier zu beanstandenden Dornfortsätze (7, 8) einer Wirbelsäule (1) einbringbar ist, dass dessen mittlerer Dämpfungsabschnitt (12) sich in diesem Zwischenraum (9) befindet, wogegen sich die Verformungsabschnitte (13, 14; 31, 32) beidseitig der Dornfortsätze (7, 8) erstrecken.

6. Implantat nach Anspruch 5, **gekennzeichnet durch** das freie Trumm (21) des aus dem zylindrischen Implantatkörper (11; 24; 24') herausragenden Stiftes (19), das geeignet ist zum Erfassen mit einer Implantatfixierungszange und vorzugsweise **durch** ein Zusammendrücken der Zangengriffe oder ein anderes mechanisches Verfahren zum Aufbau von Zugkräften, so dass die Länge des Stiftes (19) derart verkürzbar ist, dass eine ausreichende Kraft auf die Konter- (20; 27) und Druckscheibe (22; 28) in den Endbereichen der Verformungsabschnitte (13, 14; 31, 32) erzeugt wird, um diese zu den Halteflanschen (15, 16; 33, 34) zu verformten.

7. Implantat nach Anspruch 6, **dadurch gekennzeichnet, dass** die Verkürzungsstrecke des Stiftes (19) durch eine wählbare oder einstellbare Sollbruchstelle (25) wählbarer Länge an diesem begrenzt ist, wobei die Sollbruchstelle (25) die Gesamtbreite des im Endzustand implantierten Implantats (10; 10') begrenzt, und wobei die Gesamtbreite des im Endzustand implantierten Implantats (10; 10') durch ein Fixieren des Endbereichs (21') des Stiftes (19) vorzugsweise im Bereich der Sollbruchstelle (25) an der Druckscheibe (22; 28) vorgesehen ist.

8. Implantat nach einem der vorhergehenden Ansprüche, **dadurch gekennzeichnet, dass** zumindest ein Verformungsabschnitt (26) des zylindrischen Implantatskörpers (24; 24`) zum Einführen eines zylindrischen Implantierungsinstrumentes (23) in den Zwischenraum (9) zwischen den Dornfortsätzen (7, 8), in Richtung seines freien Endes verjüngt ausgebildet ist, wobei die Verjüngung als Abschnitt eines Ellipsoids oder einer sonstigen geometrischen und sich verjüngenen Form ausgebildet ist, wobei der Durchmesser der Konterscheibe (27) im Bereich der ellipsoiden Verjüngung kleiner ausgebildet ist als der Durchmesser der Druckscheibe (28) und die Konter- (29) und Druckscheibe (30) jeweils innerhalb der Verformungsabschnitte (31, 32) des zylindrischen Implantatkörpers (24') angeordnet sind und der Dämpfungsabschnitt (12) statisch oder dämpfend oder federnd bzw. elastisch ausgebildet ist.

## Claims

1. Implant for spacing adjacent bones of a spinal column, in particular the dorsally directed spinous processes arising from vertebral arches, the implant (10; 10') being configured in its initial shape as a cylindrical implant body (11; 24; 24') with a central damping section (12) and deforming sections (13, 14; 31, 32) adjoining the latter on both sides, the deforming sections (13, 14; 31; 32) being constituted in such a way that, after implantation of the initial body (11; 24; 24') between adjacent spinous processes (7; 8), they are each deformable into holding flanges (15, 16; 33, 34) according to the principle of a blind rivet for the purpose of fixing the damping section (12) to the spinous processes (7; 8), and the implant body having a hollow longitudinal axis (17), a titanium pin (19) being placed in the hollow longitudinal axis (17) with the inserted tube (18) of the implant body (11, 24), the length of which pin can be shortened in such a way that a sufficient force can be exerted on a counter-disc and pressure disc (22, 28, 29, 30) in end regions of the deforming sections (13, 14), in order that the latter are deformable into the holding flanges (15, 16) and are elastically applied against the spinous processes (7, 8),
**characterised**
**in that** a titanium tube (18) is inserted in the central region of the longitudinal axis (17), the length of which tube corresponds to the maximum outside distance between the deformed holding flanges (15, 16) in the implanted final state of the implant (10).

2. Implant according to Claim 1, **characterised in that** the cylindrical implant body (11; 24; 24') is preferably configured cylindrically, circularly or polygonally in cross-section, and **in that** the longitudinal extent of the pin (19) practically terminates, on one end side of the implant body (11; 24), at this end side and projects with an end piece (21) from the hollow longitudinal axis (17) on the other end side.

3. Implant according to Claim 2, **characterised in that** a counter-disc (20; 27) is fastened to the pin (19) on the terminating end side of the cylindrical implant body (11; 24) and bears against the end face of the latter, whereas on the end piece (21) of the pin (19) projecting from the longitudinal axis (17) of the implant body (11; 24) a pressure disc (22; 28) is displaceably arranged on the end piece (21) and bears, on this end side, against the end face of the implant body (11; 24).

4. Implant according to Claim 3, **characterised by** the cylindrical implant body (11; 24; 24'), suitable for introduction into the region (9) of a patient's spinal column (1) to be spaced, using a tubular implantation instrument (23) having in its front region the cylindrical implant body (11; 24; 24') and also a plunger (23') by which the implant body can be brought out of the tubular implantation instrument (23) into a desired position in the spinal column (1),

5. Implant according to Claim 4, **characterised in that** the cylindrical implant body (11; 24; 24') can be introduced in such a way into the interspace (9) between two spinous processes (7, 8) of a spinal column (1) which are to be spaced that its central damping section (12) is situated in this interspace (9), whereas the deforming sections (13, 14; 31, 32) extend on both sides of the spinous processes (7, 8).

6. Implant according to Claim 5, **characterised by** the free end piece (21) of the pin (19) projecting from the cylindrical implant body (11; 24; 24') which is suitable for grasping with implant fixing pliers and preferably by pressing together the handles of the pliers, or by another mechanical process, for creating tensile forces so that the length of the pin (19) can be shortened in such a way that a sufficient force is produced on the counter-disc (20; 27) and pressure disc (22; 28) in the end regions of the deforming sections (13, 14; 31, 32), in order to deform the latter into the holding flanges (15, 16; 33, 34).

7. Implant according to Claim 6, **characterised in that** the shortening distance of the pin (19) is limited by a selectable or settable predetermined breaking point (25) of selectable length thereon, the predetermined breaking point (25) limiting the overall width of the implant (10; 10') implanted in the final state, and the overall width of the implant (10; 10') implanted in the final state being provided by fixing the end region (21') of the pin (19) to the pressure disc (22; 28), preferably in the region of the predetermined breaking point (25).

8. Implant according to one of the preceding claims, **characterised in that** at least one deforming section (26) of the cylindrical implant body (24; 24') is configured in a tapering manner in the direction of its free end for the purpose of introducing a cylindrical implantation instrument (23) into the interspace (9) between the spinous processes (7, 8), the taper being configured as a section of an ellipsoid or another geometrical and tapering shape, the diameter of the counter-disc (27) being configured smaller in the region of the ellipsoid taper than the diameter of the pressure disc (28), and the counter-disc (29) and pressure disc (30) each being arranged inside the deforming sections (31, 32) of the cylindrical implant body (24'), and the damping section (12) being of static or damping or resilient or elastic design.

## Revendications

1. Implant pour éloigner des os contigus d'une colonne vertébrale, en particulier les apophyses orientées vers l'arrière partant des arcs vertébraux, l'implant (10; 10') étant réalisé, quant à sa forme de départ, sous forme de corps d'implant cylindrique (11; 24; 24') avec un segment d'atténuation central (12) et des segments de déformation (13, 14; 31, 32) se raccordant des deux côtés à ce dernier, les segments de déformation (13, 14; 31; 32) étant tels qu'après une implantation du corps de départ (11; 24; 24') entre des apophyses contiguës (7; 8), ils soient, pour la fixation du segment d'atténuation (12) aux apophyses (7; 8), chaque fois déformables, pour former des brides de retenue (15, 16; 33, 34) selon le principe d'un rivet borgne, et le corps d'implant présentant un axe longitudinal creux (17), dans l'axe longitudinal creux (17) avec le tube (18) du corps d'implant (11, 24) introduit étant engagée une goupille en titane (19) dont la longueur peut être raccourcie de sorte que puisse être exercée une force suffisante sur une rondelle de fixation et une rondelle de pression (22, 28, 29, 30) dans les zones d'extrémité des segments de déformation (13, 14) pour qu'ils soient déformables pour former des brides de retenue (15, 16) et qu'ils s'appliquent de manière élastique contre les apophyses (7, 8),
**caractérisé par le fait**
**que** dans leur zone centrale de l'axe longitudinal (17) est introduit un tube en titane (18) dont la longueur correspond à la distance extérieure maximale entre les brides de retenue déformées (15, 16) à l'état final implanté de l'implant (10).

2. Implant selon la revendication 1, **caractérisé par le fait que** le corps d'implant cylindrique (11; 24; 24') est réalisé de préférence à section cylindrique, circulaire ou carrée et que l'extension longitudinale de la goupille (19), d'un côté d'extrémité du corps d'implant (11; 24), se termine pratiquement à ras avec ce dernier et, de l'autre côté d'extrémité, ressort de l'axe longitudinal creux (17) par un bout (21).

3. Implant selon la revendication 2, **caractérisé par le fait qu'**à la goupille (19) est fixée, du côté d'extrémité à ras du corps d'implant cylindrique (11; 24), une rondelle de fixation (20; 27), tandis que sur le bout (21) de la goupille (19) ressortant de l'axe longitudinal (17) du corps d'implant (11; 24) est disposée de manière déplaçable sur le bout (21), une rondelle de pression (22; 28) en applique contre le côté frontal du corps d'implant (11; 24) de ce côté d'extrémité.

4. Implant selon la revendication 3, **caractérisé par** le corps d'implant cylindrique (11; 24; 24') convenant pour introduire dans la zone à éloigner (9) de la colonne vertébrale (1) d'un patient par un instrument d'implantation tubulaire (23) présentant, dans sa zone frontale, le corps d'implant cylindrique (11; 24; 24') ainsi qu'un poinçon (23') par lequel le corps d'implant peut être introduit, à partir de l'instrument d'implantation tubulaire (23), en une position désirée dans la colonne vertébrale (1).

5. Implant selon la revendication 4, **caractérisé par le fait que** le corps d'implant cylindrique (11; 24; 24') peut être introduit dans l'espace intermédiaire (9) entre deux apophyses (7, 8) à éloigner d'une colonne vertébrale (1) de sorte que son segment d'atténuation central (12) se trouve dans cet espace intermédiaire (9), tandis que les segments de déformation (13, 14; 31, 32) s'étendent des deux côtés des apophyses (7, 8).

6. Implant selon la revendication 5, **caractérisé par** le bout libre (21) de la goupille (19) sortant du corps d'implant cylindrique (11; 24; 24'), lequel convient pour être saisi par une pince de fixation d'implant et, de préférence, par une compression des poignées de pince ou un autre procédé mécanique pour créer des forces de traction, de sorte que la longueur de la goupille (19) puisse être raccourcie de sorte que soit générée une force suffisante sur la rondelle de fixation (20; 27) et la rondelle de pression (22; 28) dans les zones d'extrémité des segments de déformation (13, 14; 31, 32), pour les déformer, pour obtenir les brides de retenue (15, 6; 33, 34).

7. Implant selon la revendication 6, **caractérisé par le fait que** le trajet de raccourcissement de la goupille (19) est limité par un point de rupture de consigne (25) sélectionnable ou réglable de longueur sélectionnable sur ce dernier, le point de rupture de consigne (25) limitant la largeur totale de l'implant (10; 10') implanté à l'état final, et la largeur totale de l'implant (10; 10') implanté à l'état final étant prévue par une fixation de la zone d'extrémité (21') de la goupille (19) de préférence à l'endroit du point de rupture de consigne (25) à la rondelle de pression (22; 28).

8. Implant selon l'une des revendications précédentes, **caractérisé par le fait qu'**au moins un segment de déformation (26) du corps d'implant cylindrique (24; 24') est réalisé de manière effilée en direction de son extrémité libre, pour introduire l'instrument d'implantation cylindrique (23) dans l'espace intermédiaire (9) entre les apophyses (7, 8), l'effilement étant réalisé sous forme de segment d'une ellipsoïde ou d'une autre forme géométrique et s'effilant, le diamètre de la rondelle de fixation (27) étant réalisé à l'endroit de l'effilement ellipsoïde plus petit que le diamètre de la rondelle de poussée (28), et la rondelle de fixation (29) et la rondelle de poussée (30) étant, chacune, disposées dans les segments de déformation (31, 32) du corps d'implant cylindrique (24') et le segment d'atténuation (12) étant réalisé de manière statique ou amortissante ou à effet de ressort ou élastique.
